# EUROPEAN PATENT APPLICATION

(11) **EP 2 808 726 A2**
(43) Date of publication of application: **03.12.2014**
(21) Application number: 14160072.6
(22) Date of filing: 14.03.2014
(51) Int. Cl.: G02C 7/04, G02B 27/00

(54) **Method and ophthalmic device for providing visual representations to a user**

(30) Priority: 15.03.2013 US 201361801960 P; 17.05.2013 US 201313896914
(71) Applicant: Johnson & Johnson Vision Care, Inc., Jacksonville, FL 32256 (US)
(72) Inventor: Pugh, Randall B., St. Johns FL Florida 32259 (US); Flitsch, Frederick A., New Windsor NY New York 12553 (US)
(74) Representative: Carpmaels & Ransford LLP

(57) **Abstract**

An energized ophthalmic lens device is disclosed. The energized ophthalmic lens device can include one or more modulated photonic emitters, a media insert supporting a first processor and one or more light sources, and one or more antennas configured to communicate with the first processor and a second processor. The one or more light sources can be configured to generate light. At least a portion of the generated light from the one or more light sources can be emitted by the one or more photonic emitters. The first processor can be configured to receive, from a sensor, an indication to project a visual representation. The processor can further be configured to control, in response to the received indication, at least one of the one of more modulated photonic emitters and the one or more light sources based on one or more programmed parameters.

## Description

### FIELD OF THE DISCLOSURE

The disclosure generally related to an energized ophthalmic device and associated method for providing visual representations to a user. In particular, the visual representations which are projected based on a sensed response and/or transmitted data received by the ophthalmic device.

### BACKGROUND OF THE DISCLOSURE

Traditionally, an ophthalmic device, such as a contact lens, an intraocular lens, or a punctal plug, included a biocompatible device with a corrective, cosmetic, or therapeutic quality. A contact lens, for example, may provide one or more of vision correcting functionality, cosmetic enhancement, and therapeutic effects. Each function is provided by a physical characteristic of the lens. A design incorporating a refractive quality into a lens may provide a vision corrective function. A pigment incorporated into the lens may provide a cosmetic enhancement. An active agent incorporated into a lens may provide a therapeutic functionality. Such physical characteristics are accomplished without the lens entering into an energized state. A punctal plug has traditionally been a passive device.

Recently, active energized ophthalmic devices have been developed. It has been theorized from development efforts resulting from these that said energized ophthalmic devices can be capable of containing light source elements that may be useful to project images. Projected images would include text and superimposed images on a user's normal sight. In addition to envisioning use, however, many limitations must be resolved in order for such light sources to function in a useful manner and do it safely.

As a consequence of the foregoing, a need exist for methods and ophthalmic devices that can overcome volume limitations and provide visual representations in useful and safe manners.

### SUMMARY OF THE DISCLOSURE

Accordingly, the foregoing needs are met, to a great extent, by the present disclosure, wherein in one aspect a Media Insert with Photonic Emitters that may be included in an Energized Ophthalmic Device, and in some embodiments, specifically, a contact lens are disclosed. The Photonic Emitters may provide light patterns forming Visual Representations including, for example, signals, image enhancements, and/or dynamic images from said light patterns that can be used to convey a message to a user.

An energized ophthalmic lens device is disclosed. The energized ophthalmic lens device can include one or more modulated photonic emitters, a media insert supporting a first processor and one or more light sources, and one or more antennas configured to communicate with the first processor and a second processor. The one or more light sources can be configured to generate light. At least a portion of the generated light from the one or more light sources can be emitted by the one or more photonic emitters. The first processor can be configured to receive, from a sensor, an indication to project a visual representation. The processor can further be configured to control, in response to the received indication, at least one of the one of more modulated photonic emitters and the one or more light sources based on one or more programmed parameters. The second processor can be configured to generate the Visual Representation.

In some aspects of the disclosure, these components may all be assembled in an Ophthalmic device that may have a size and shape that is consistent with the Ophthalmic device occupying a position that is between a user's eye surface and a that eye's respective eye lid.

An Ophthalmic device may be formed comprising a projection system along with energization elements, control circuitry, communication circuitry and data processing circuitry into a single entity. The projection system may be made up of a subsystem comprising at least a Photonic Emitter element, a light source, a light modulating element and a lens element. The projection systems may also be made up of subsystems that comprise combinations of Photonic Emitter elements and an associated Pixel Based Light Modulating Elements.

An ophthalmic device, which incorporates a projection system, may display data or information in various forms. The display may project text-based information. Similarly, the display may project images. The images may be of the form of digital images comprised of multiple pixels of image data projected. The images may be displayed as a monochrome display or alternatively have various degrees of color. By altering the display on a time scale, the projection system may display data in the form of video of various formats.

The exemplary display of an ophthalmic display comprising a system of Photonic Emitters may incorporate lenses as part of the ophthalmic device. These lenses may act on the image formed from the system of photonic emitters and focus that image in various ways onto the user's retina. The far field image created by the array of photonic emitters or the near field image created by the array of photonic emitters may be focused by the lens system. In some embodiments, the lens system may comprise multiple lens subsystems. In some embodiments, the lens subsystems may have elements that have a fixed focal characteristic or a fixed focal length. In other embodiments, the lens subsystem may include at least a first variable focal length lens. An example of such a variable focal length lens may include a meniscus-based lens that may also function utilizing the EWOD effect. Complex variable focal length lens may also be formed with multiple electrode regions that may be useful to move the focal point characteristic of the lens both from a focal length perspective but also from a translational perspective that may effectively vary where the image is projected. In some cases, the image may be projected by the system through a user's eye and upon a user's retina. When projected on the user's retina, the size of the image formed by the extent of the imaged photonic elements may be less than a square centimeter in size. In other embodiments the size may be less than or approximately equal to a square millimeter in size.

There has thus been outlined, rather broadly, certain aspects of the disclosure in order that the detailed description herein may be better understood, and in order that the present contribution to the art may be better appreciated.

In this respect, before explaining at least one embodiment of the disclosure in detail, it is to be understood that the disclosure is not limited in its application to the details of the construction and to the arrangements of the components set forth in the following description or illustrated in the drawings. The disclosure is capable of embodiments in addition to those described and of being practiced and carried out in various ways. Also, it is to be understood that the phraseology and terminology employed herein, as well as the abstract, are for the purpose of description and should not be regarded as limiting.

As such, those skilled in the art will appreciate that the conception upon which this disclosure is based may readily be utilized as a basis for the designing of other devices and systems for carrying out the several purposes of the present disclosure. It is important, therefore, that the claims be regarded as including such equivalent constructions insofar as they do not depart from the spirit and scope of the present disclosure.

### BRIEF DESCRIPTION OF THE DRAWINGS

Fig. 1 illustrates an isometric view of an exemplary ophthalmic lens 150 with a cross sectional cut out and a top view of the Media Insert 100 implemented according to aspects of the present disclosure.
Fig. 2 illustrates a top view A and a cross sectional view B of an exemplary multi-piece Media Insert 200 according to aspects of the present disclosure.
Fig. 3 illustrates a top view A and a cross sectional view B of another exemplary alternative embodiment to that demonstrated in Fig. 2 wherein the Media Insert comprises an active focal adjusting lens system.
Fig. 4 illustrates exemplary Photonic Emitter structures, which may be included in some embodiments of the present invention.
Fig. 5 illustrates an exemplary array structure 500 of Photonic Emitters pixels 520 with a light source 560 and means of coupling the light source to the array.
Fig. 6 illustrates an exemplary device comprising an array of Photonic Emitters within a portion of the optical zone of an exemplary ophthalmic device.
Fig. 7 illustrates an exemplary light modulating element structure according to some aspects of the disclosure.
Fig. 8 illustrates an alternative exemplary light modulating element structure that may be useful for implementing some aspects of the disclosure.
Fig. 9 illustrates an exemplary energized ophthalmic device 900 for a projection system comprising photonic arrays, light phase or intensity modulation arrays and lens systems that may be useful for implementing some aspects of the disclosure.
Fig. 10 illustrates method steps related to the use of ophthalmic devices comprising Photonic Emitters according to some aspects of the disclosure.
Fig. 11 illustrates a perspective view of a geographic setting with objects useful for a sensor and a processor to associate related data to provide Visual Representations.
Fig. 12 illustrates a block diagram of processor apparatus that may be used to implement various aspects of the present disclosure.
Fig. 13 illustrates method steps related to the projection of Visual Representations according to some aspects of the disclosure.

### DETAILED DESCRIPTION OF THE INVENTION

### GLOSSARY

In this description and claims directed to the presented invention, various terms may be used for which the following definitions will apply:

Electro-wetting on Dielectric or EWOD: as used herein refers to a class of devices or a class of portions of devices where a combination of immiscible fluids or liquids, a surface region with defined surface free energy and an electro-potential field are present. Typically, the electro-potential field will alter the surface free energy of the surface region, which may alter the interaction of the immiscible fluids with the surface region.

Energized: as used herein refers to the state of being able to supply electrical current to or to have electrical energy stored within.

Energy: as used herein refers to the capacity of a physical system to do work. Many uses within this invention may relate to the said capacity being able to perform electrical actions in doing work.

Energy Source: as used herein refers to a device or layer that is capable of supplying Energy or placing a logical or electrical device in an Energized state.

Energy Harvester: as used herein refers to a device capable of extracting energy from the environment and converting it to electrical energy.

Functionalized: as used herein refers to making a layer or device able to perform a function including for example, energization, activation, or control.

Leakage: as used herein refers to unwanted loss of energy.

Lens or Ophthalmic Device: as used herein refers to any device that resides in or on the eye. These devices may provide optical correction, may be cosmetic, or may provide functionality unrelated to the eye. For example, the term lens may refer to a contact lens, intraocular lens, overlay lens, ocular insert, optical insert, or other similar device through which vision is corrected or modified, or through which eye physiology is cosmetically enhanced (e.g. iris color) without impeding vision. Alternatively, the Lens may provide non-optic functions such as, for example, monitoring glucose or administrating medicine. In some embodiments, the preferred lenses of the invention are soft contact lenses are made from silicone elastomers or hydrogels, which include, for example, silicone hydrogels, and fluorohydrogels.

Lens-forming mixture or Reactive Mixture or Reactive Monomer Mixture (RMM): as used herein refers to a monomer or prepolymer material that may be cured and crosslinked or crosslinked to form an ophthalmic lens. Various embodiments may include lens-forming mixtures with one or more additives such as, for example, UV blockers, tints, photoinitiators or catalysts, and other additives one might desire in an ophthalmic lenses such as, contact or intraocular lenses.

Lens-forming Surface: as used herein refers to a surface that is used to mold a lens. In some embodiments, any such surface can have an optical quality surface finish, which indicates that it is sufficiently smooth and formed so that a lens surface fashioned by the polymerization of a lens forming material in contact with the molding surface is optically acceptable. Further, in some embodiments, the lens-forming surface can have a geometry that is necessary to impart to the lens surface the desired optical characteristics, including without limitation, spherical, aspherical and cylinder power, wave front aberration correction, corneal topography correction and the like as well as any combinations thereof.

Light Modulating Element: as used herein refers to a device or portion of a device that modulates the intensity of light transmitting from one side to another. The ideal light modulating elements in embodiments herein can be able to transmit all light in one state and no light in another. Practical elements may substantially achieve the ideal aspects.

Lithium Ion Cell: as used herein refers to an electrochemical cell where Lithium ions move through the cell to generate electrical energy. This electrochemical cell, typically called a battery, may be reenergized or recharged in its typical forms.

Media Insert: as used herein refers to an encapsulated insert that will be included as part of an energized ophthalmic device. The energization elements and circuitry may be incorporated in the Media Insert. The Media Insert defines the primary purpose of the energized ophthalmic device. For example, in embodiments where the energized ophthalmic device allows the user to adjust the optic power, the Media Insert may include energization elements that control a liquid meniscus portion in the Optical Zone. Alternatively, a Media Insert may be annular so that the Optical Zone is void of material. In such embodiments, the energized function of the Lens may not be optic quality but may be, for example, monitoring glucose or administering medicine.

Operating Mode: as used herein refers to a high current draw state where the current over a circuit allows the device to perform its primary energized function.

Optical Zone: as used herein refers to an area of an ophthalmic lens through which a wearer of the ophthalmic lens sees.

Photonic Emitter: as used herein refers to a device or device portion that may receive incident light and transmit that light into free space. The light may typically proceed in an altered direction than that incident upon the emitter. The Emitter may typically comprise an antenna structure to transmit the light.

Pixel Based Light Modulation System: as used herein refers to a combination of light modulating elements that function individually wherein each individually function portion of the light modulation system may be considered a pixel or picture element.

Power: as used herein refers to work done or energy transferred per unit of time.

Rechargeable or Re-energizable: as used herein refers to a capability of being restored to a state with higher capacity to do work. Many uses within this invention may relate to the capability of being restored with the ability to flow electrical current at a certain rate and for a certain, reestablished period.

Reenergize or Recharge: as used herein refers to restoring to a state with higher capacity to do work. Many uses within this invention may relate to restoring a device to the capability to flow electrical current at a certain rate and for a certain, reestablished period.

Reference: as use herein refers to a circuit which produces an, ideally, fixed and stable voltage or current output suitable for use in other circuits. A reference may be derived from a bandgap, may be compensated for temperature, supply, and process variation, and may be tailored specifically to a particular application-specific integrated circuit (ASIC).

Reset Function: as used herein refers to a self-triggering algorithmic mechanism to set a circuit to a specific predetermined state, including, for example, logic state or an energization state. A Reset Function may include, for example, a power-on reset circuit, which may work in conjunction with the Switching Mechanism to ensure proper bring-up of the chip, both on initial connection to the power source and on wakeup from Storage Mode.

Sleep Mode or Standby Mode: as used herein refers to a low current draw state of an energized device after the Switching Mechanism has been closed that allows for energy conservation when Operating Mode is not required.

Stacked: as used herein means to place at least two component layers in proximity to each other such that at least a portion of one surface of one of the layers contacts a first surface of a second layer. In some embodiments, a film, whether for adhesion or other functions may reside between the two layers that are in contact with each other through said film.

Stacked Integrated Component Devices or SIC Devices: as used herein refers to the products of packaging technologies that assemble thin layers of substrates that may contain electrical and electromechanical devices into operative-integrated devices by means of stacking at least a portion of each layer upon each other. The layers may comprise component devices of various types, materials, shapes, and sizes. Furthermore, the layers may be made of various device production technologies to fit and assume various contours.

Storage Mode: as used herein refers to a state of a system comprising electronic components where a power source is supplying or is required to supply a minimal designed load current. This term is not interchangeable with Standby Mode.

Substrate Insert: as used herein refers to a formable or rigid substrate capable of supporting an Energy Source within an ophthalmic lens. In some embodiments, the Substrate insert also supports one or more components.

Switching Mechanism: as used herein refers to a component integrated with the circuit providing various levels of resistance that may be responsive to an outside stimulus, which is independent of the ophthalmic device.

### ENERGIZED OPHTHALMIC DEVICE

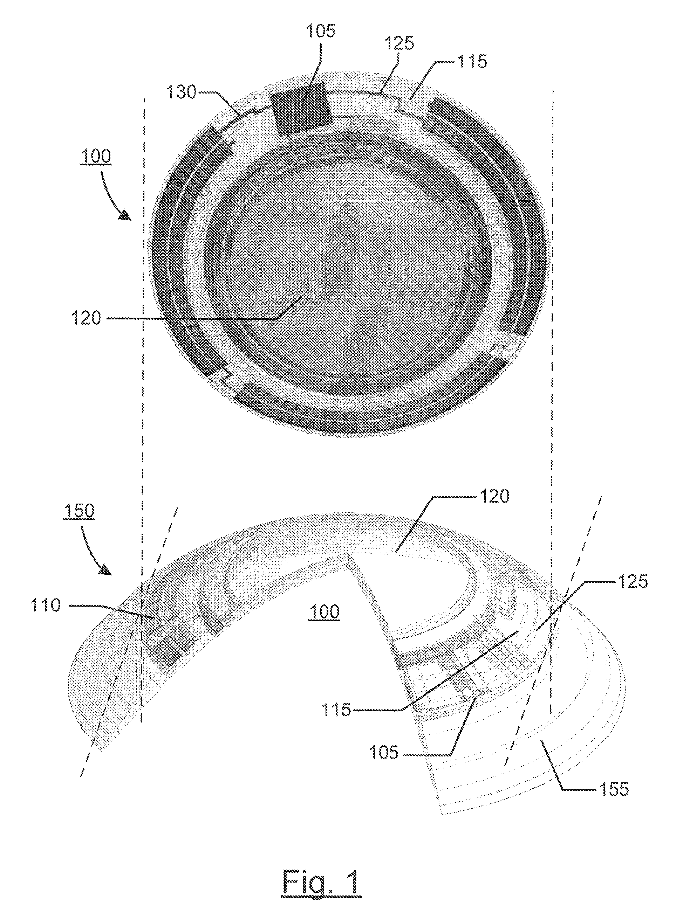

Starting at Fig. 1, an isometric view of an exemplary Ophthalmic Lens 150 with a cross sectional cut out and a top view of the Media Insert 100 implemented according to aspects of the present disclosure are illustrated. The Media Insert 100 may comprise an Optical Zone 120 that may or may not be Functional to provide vision correction. Where the energized function of the Ophthalmic Lens 150 is unrelated to vision, the Optical Zone 120 of the Media Insert 100 may be void of material. In some embodiments, the Media Insert 100 may include a portion not in the Optical Zone 120 comprising a Substrate Insert 115 incorporated with Energization elements 110 and electronic components 105. According to aspects of the disclosure, electronic components may include numerous embodiments relating to Photonic Emitters as further described in subsequent parts of the disclosure.

In some embodiments, a power source 110, such as a battery and a load, which can be, for example, a semiconductor die, may be attached to the substrate 115. Conductive traces 125 and 130 may electrically interconnect the electronic components 105 and the Energization elements 110. The Media Insert 100 may be encapsulated to protect and contain the Energization elements 110, traces 125 and 130, and electronic components 105. In some embodiments, the encapsulating material may be semi-permeable, for example, to prevent specific substances, such as water, from entering the Media Insert 100 and to allow specific substances, such as ambient gasses or the byproducts of reactions within Energization elements 110, to penetrate or escape from the Media Insert 100.

As depicted, in some embodiments the Media Insert 100 may be included in an Ophthalmic Device 150, which may comprise a polymeric biocompatible material. The Ophthalmic Device 150 may include a rigid center, soft skirt design wherein a central rigid optical element comprises the Media Insert 100. In some specific embodiments, the Media Insert 100 may be in direct contact with the atmosphere and/or the corneal surface on respective anterior and posterior surfaces, or alternatively, the Media Insert 100 may be encapsulated in the Ophthalmic Device 150. The periphery 155 of the Ophthalmic Device 150 may be a soft skirt material, including, for example, a hydrogel material.

The infrastructure of the Media Insert 100 and the Ophthalmic Device 150 may provide an environment for numerous embodiments involving light projection with Photonic Emitters, which may be combined with active or non-active lens devices and in some embodiments with light intensity modulating arrays. Some of these embodiments may involve purely passive function of the portion of the Ophthalmic Device 150 not related to the photonic projection components. Other embodiments, may involve the Ophthalmic Device 150 having active functions that may complement or supplement the function of the photonic projection components. For example, the non-projection portions of the device may provide vision correction or active "screening" of the device such that its transparency to incident light may be reduced.

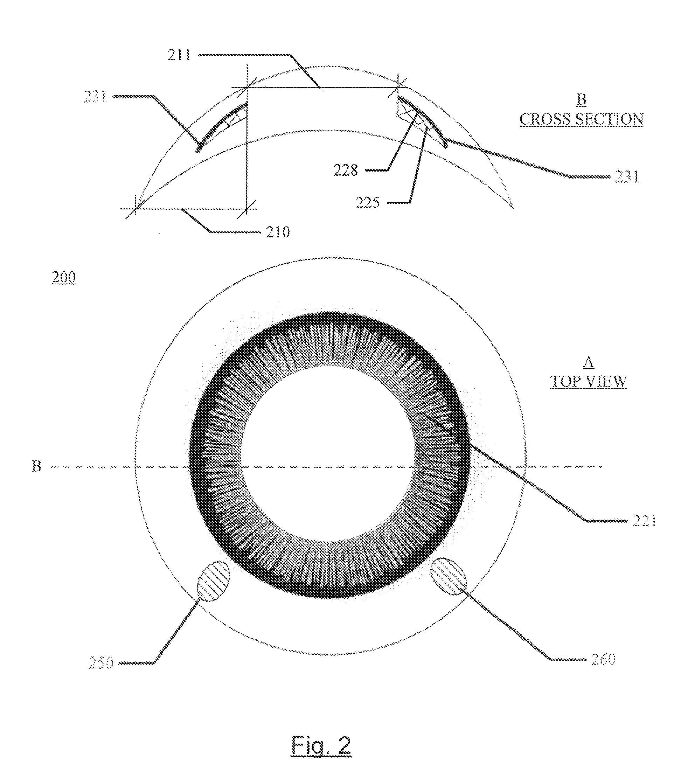

Referring now to Fig. 2, a top view representation A and a cross section representation B of an exemplary multi-piece Media Insert 200 are illustrated. The multi-piece Media Insert 200 of this type can be an annular insert with a ring of material around a central optical zone 211 that may be devoid of material. In some embodiments, the peripheral zone 210 region of the insert outside the optic zone 211 may include Energization elements 225 and controlling electronics 228 to support active elements 231 of various kinds. These active elements 231 may typically include sensors and communication elements. In addition, elements to provide the control and energization function for a projection element (not shown) based upon photonic projection elements can be included. As well, outside the optic zone 211 of the device there may be printed patterns 221 placed on the Media Insert 200.

In some embodiments, there may be a requirement for orientation of the Ophthalmic Lens within the ocular environment. Stabilization zone features 250 and 260 may be included and can aid in orienting the formed Ophthalmic Device upon a user's eye. Moreover, in some embodiments the use of orientation features (not shown) upon the multi-piece annular Media Insert 200 may allow for its orientation relative to the molded stabilization features 250 and 260, which may be particularly important for placements of projection elements and lens systems that do not have dynamic focus and centering controls.

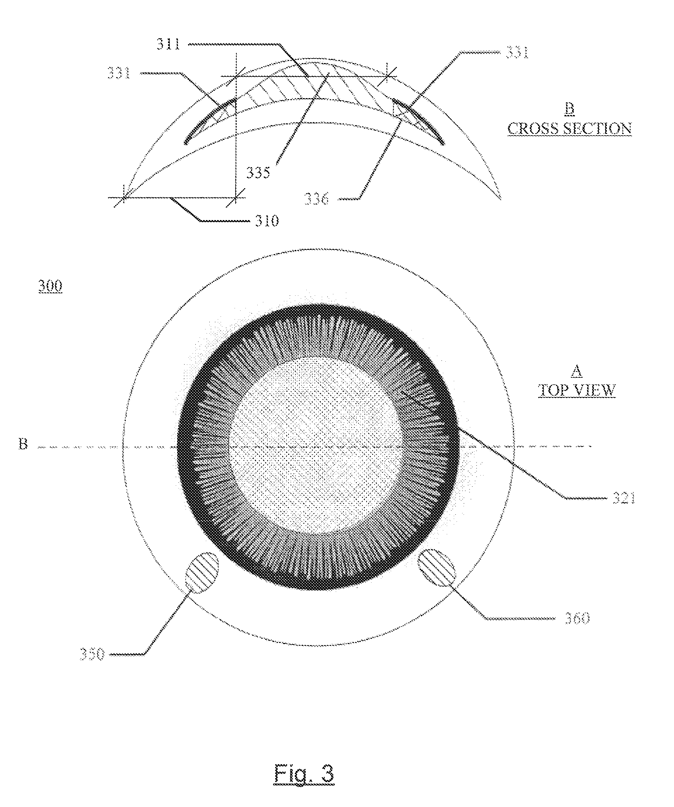

Referring now to Fig. 3, a top view A and a cross sectional view B of another exemplary alternative embodiment to that demonstrated in Fig. 2 wherein the Media Insert 300 comprises an active focal adjusting lens system 335 is illustrated. The Optical Zone 311 of the Ophthalmic Device may include a portion where an active focal adjusting lens system 335 such as a liquid meniscus based lens system may be found. In the periphery 310, outside the optic zone 311 of the Media Insert 300 there may be portions of the insert that contain energization elements 336 and control and activation components 331. For similar motivations as the embodiment in Fig. 2, there may be alignment features and/or stabilization zones 350 and 360 incorporated into the Ophthalmic Device, and there may be patterns printed upon the insert as features 321.

### PHOTONIC PROJECTION ELEMENTS

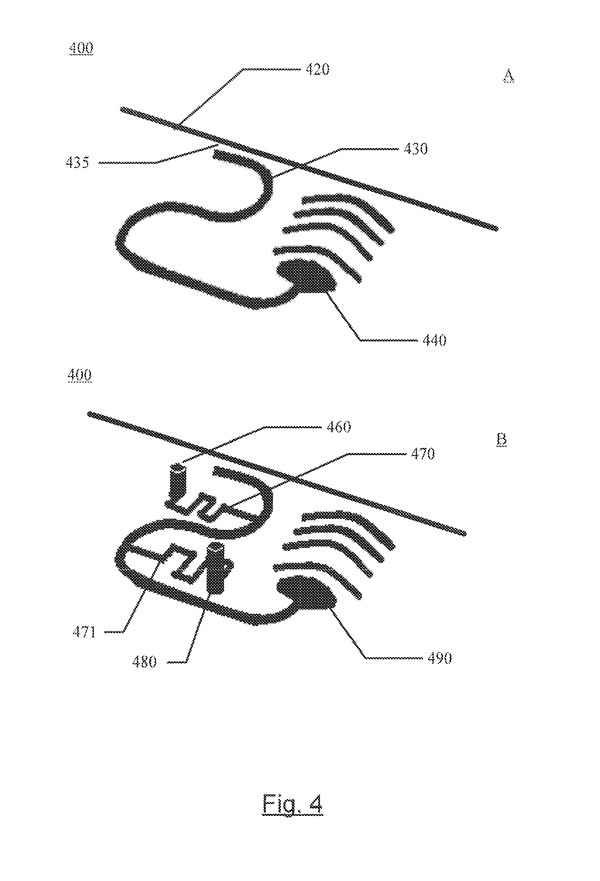

Referring now to Fig. 4, exemplary Photonic Emitter 400 structures A and B, which may be included in some embodiments of the present disclosure are illustrated. There may be numerous manners of defining emitter (which may also be considered radiator) elements for use with photonic applications. A Photonic Emitter 400, at A demonstrates a simple Photonic Emitter element. The source of the photons for the system may be a light pipe 420 that runs parallel to coupling portions 430 of the radiator element. Photons travelling through the light pipe 420 may couple to the coupling portions 430 by a process which may be called evanescent coupling; an exponentially decaying phenomena in the near region to the periphery of the light pipe. The coupling of the coupling portions 430 can allow photons to move from the light pipe 420 to the radiator element 440. The degree of the coupling and therefore the number of photons that enter the radiator element 440, which is a type of intensity, may be modulated by a number of phenomena such as the materials used, the ambient conditions but more importantly the structural design of the system. The length of the parallel portion of coupling portions 430 and the gap 435 between this region and the light pipe may dominate the efficiency of coupling and can be used to adjust the nominal relative intensity of a Photonic Emitter 400 in a collection of Photonic Emitters. For example, in Photonic Emitter A, the light will proceed through the element's light guiding components in the coupling portion 430 until it reaches the radiator portion 440, shaped in a diffraction grating. Numerous effects can be exploited to increase the efficiency of light through the Photonic Emitter 400, as for example the constructed angle of the emission surfaces and their shape and gap dimension. Ideally as much light as possible will be emitted at the radiator element 440 in one direction, for example "out of the page."

At Photonic Emitter B, a more sophisticated Photonic Emitter 400 may be found. A heating mechanism may be incorporated into the emitter cell. The heating mechanism may be comprised of a resistive heater built into the Photonic Emitter 400. In embodiments, where the emitter is formed in semiconducting materials, like silicon, the resistor may be formed in the same layer where it may be doped to alter resistivity characteristics. By flowing a current from a contact 480, through a resistive arm 470, and through a portion of the emitter body 430 and back through another portion of the resistive arm 471 and through a contact 460, the Photonic Emitter 400 may have a portion of the light path differentially heated. Thermal effects in light pipes such as A, may alter the phase characteristics of the light that travels through them. Thus, the Photonic Emitter 400 depicted at B may have a certain intensity of light emitted from it based on the intensity in the source light pipe 420 and the efficiency of coupling of source light into the radiation element 490 based on the proximity of a coupling region of the emitter device and the dimensions of that coupling region. Moreover, in addition the phase of that light may be controllably altered based on the application of an electrical current through the heater portion between resistive arm 460 and resistive arm 480. Control of the relative phase of emitted light in such a manner may result in the effective transmission of information encoded in the phase characteristics being observable in the far field image of an array built with such Photonic Emitter 400 where the phase of individual pixels may be controlled by the thermal state imposed on portions of the emitter device. Accordingly, there may be numerous materials that such a Photonic Emitter 400 may be constructed in and there may be numerous means for different materials to introduce phase effects including thermal controls and mechanical stress controls as non-limiting examples.

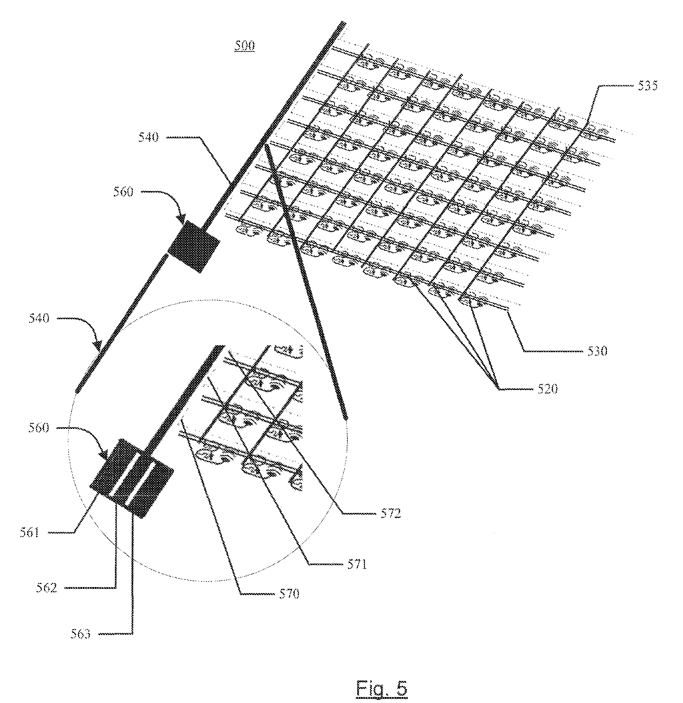

Referring now to Fig. 5, an exemplary array structure 500 constructed from Photonic Emitters pixels 520 with a light source 560 and means of coupling the light source to the array is depicted. In some embodiments, the Photonic Emitter pixels 520 may be defined in a similar fashion to the Photonic Emitters illustrated in Fig. 4. Light can be supplied using a light source 560 which, in some embodiments, may be comprised of one or more laser elements 561, 562 and 563 emitting light into one or more supply light pipes 540 for the Photonic Emitter array structure 500. Electrical current flowing through the heated portions of a pixel 520 may be introduced by conductive metal lines built into the Photonic Emitter array structure 500 in similar fashions to the metal lines in an integrated circuit. A set of word lines 530 may have corresponding bit lines 535 to allow the addressing of individual cells in an efficient fashion. In some embodiments, the photonic array structure 500 may be built into the silicon substrate useful to construct control electronics for the array itself. The exemplary Photonic Emitter pixels 520 may have a dimension about 9 microns by 9 microns or smaller. Thus, an array of 64 x 64 emitters may have a scale of roughly 0.5mm by 0.5 mm in size. The actual dimensions of the Photonic Emitter pixels 520 may vary in a matrix and may be different for different targeted wavelengths of emission.

In the inset 550 of the array structure 500, a close up version of the light source 560 and the supply light pipe or pipes 540 is shown. Light from a light source 560 may be guided into the light pipe 540. Along the dimension of the light pipe 540, additional distribution elements in the form of additional light pipes may be found. In some embodiments, for example, light pipes 570, 571 and 572 can be coupled into the main supply light pipe 540 and run roughly perpendicular to distribute light to rows of Photonic Emitter pixels 520. The design aspects of the pipes and the individual Photonic Emitter pixels 520 along the row may be optimized for each element so that a particular intensity pattern along the row and in the array structure 500 may be obtained. In a preferred example, the array structure 500 may be designed such that the resulting emission intensity from each pixel is approximately the same for all elements.

In some embodiments, multiple light sources 561, 562 and 563 at different wavelengths may be used to impart light on a single source light pipe 540 or in some embodiments; the light pipe 540 may be comprised of multiple pipes. In the example, there may be three different light sources 561, 562 and 563. Where in a non-limiting example source 561 may comprise a red light source, source 562 may comprise a green light source and 563 may comprise a blue light source. There may be numerous types of sources of light consistent with the inventive art including solid state lasers, or solid state light emitting diodes, or filtered incandescent lamps as non-limiting examples. In embodiments where the relative phase of the pixels in the array may be important for encoding information, the light source may be characterized by a desired coherence of the light output. Other embodiments may function with non-coherent light sources.

If there are multiple wavelengths provided in the supply source, the interaction of the rows of light pipes shown as item 570 may be controlled so that one light source is favored for a particular row. This may be controlled by the use of filtering materials in the region where the light pipe for a row 570 couples to the supply light pipe. Alternatively, if there are multiple supply light pipes, the pipes for the non-desired wavelengths for a particular light source may be blocked by absorbing material. There may be numerous materials that may be used to block the light coupling including metallic materials or the use of heavy doping levels in a semiconductor material.

In an alternative embodiment, the multiple light sources 561, 562, and 563 may have a duty cycle. They may be turned on or off for their turn to use the source light pipe(s) 540. In such an embodiment, there may not be a need for either multiple source lines or controls to funnel different light sources to different regions of the array structure 500. However, the design of the Photonic Emitter pixels 520 may have to be performed in such a manner that is not optimized for a particular wavelength but optimized for all wavelengths employed. In some embodiments, the Photonic Emitter pixels 520 may be comprised by multiple emitters where one of the Photonic Emitters pixels 520 may be optimized for a particular source.

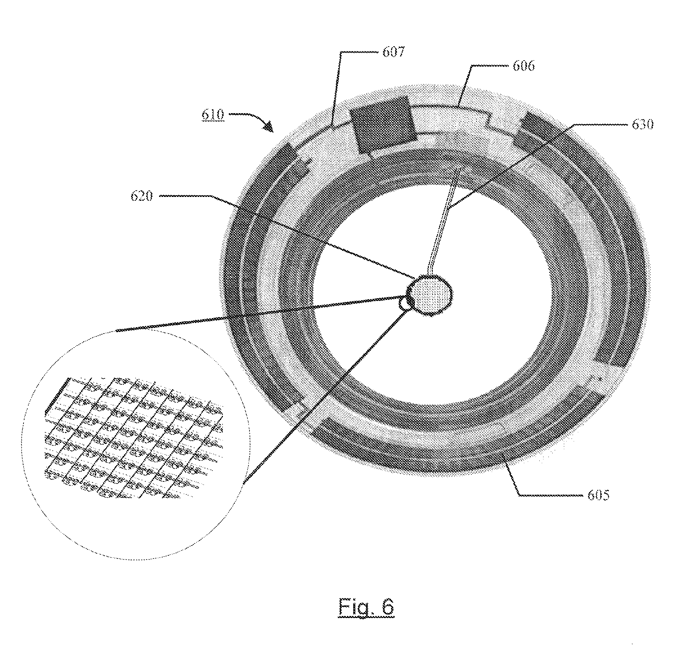

In the array structure 500 where the individual pixels include phase shifting components within their design, it may be useful to include lenses (not shown) that allow for the focusing of the far field image of the array onto a particular point, which may include a user's retina. In a single light source embodiment, it may be important for coherent light to be used as the source. The resulting far field image may comprise an image constructed from the phase information within the individual pixels. An example of such an embodiment where a photonic array projecting far field phase controlled pixel images is illustrated in Fig. 6. As previously described, the ophthalmic lens Media Insert 610 may contain energization elements 605, and control circuitry 606 and 607 to control electrical signals through an electrical bus 630. In some embodiments, this electrical bus 630 may be constructed of conductors having minimum visible light absorbance characteristics. For example, Indium Tin Oxide (ITO) may be used.

A projection system 620 may be located at or near the center of the optical zone, and may comprise an array of Photonic Emitters as previously described and shown in Fig. 5 along with control circuitry, light sources, and lens elements among other included components. An alternative embodiment may involve the use of the photonic array as an emitter of light where the phase characteristics are not the primary focus.

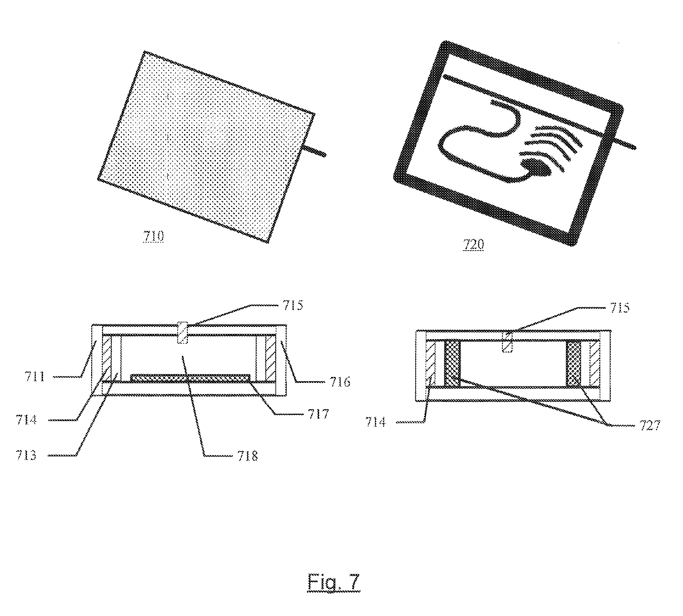

Referring now to Fig. 7, an exemplary light modulating element structure according to some aspects of the disclosure is illustrated. A pixel element 720 utilizing the exemplary Photonic Emitter without an incorporated heater may be used. In some embodiments, the incorporation of the heater may still be desirable. For example, when the near field image of the resulting array is focused on a particular position, the light source may be part of a projection system where each pixel has an element that controls the transmitted intensity that proceeds from the emitter to the user's retina. One example of a light intensity-controlling element aligned to each photonic emission element is illustrated at Fig. 7.

The phenomena of Electro-wetting on Dielectrics may be used to control intensity transmitted on a pixel-by-pixel basis. The technique can act on combinations of liquids by changing the surface free energy of surfaces near the liquids. Combinations of immiscible liquids, where one liquid, for example is a polar liquid aqueous solution, and the other liquid is a non-polar oil, they may be effective for EWOD devices. One of these liquids may be formulated to be transparent to light in a particular desired wavelength regime whereas the other liquid may be opaque at those or all visible wavelengths. The liquid itself may have such properties, or the liquid may be combined with dying agents to result in the desired wavelength blocking effect. In addition, it may be possible to include different combinations of liquids with different inherent wavelength blocking capabilities in different pixel elements in the same device.

In an example embodiment, an oil based non-aqueous liquid may comprise a dying agent to render an effective absorbance in a layer of an EWOD pixel cell that may be considered a Light Modulating Element. For example, pixel element 710 where the oil-based liquid is located across the pixel can be able to absorb significant quantities of light. There may be isolation structure 711 and 716 that define the edges of the pixel cell with the oil-based liquid 717 and the aqueous fluid 718. A coating 713 with a material that has a surface free energy such that it may repel oil-based fluids can be utilized. Therefore in a standard non energized state, the fluids would prefer to assume a location where the dyed oil based phase can be localized across the interior region of the pixel away from surface 713, and therefore in the light path of light proceeding through the pixel. A combination of electrodes 715 and 714 along with a dielectric underlying or comprising the material of surface 713 can allow for an application of an electro-potential across the two immiscible liquids. By applying an electro-potential across the electrodes, the free energy of surface 713 may be altered to attract the oil-based liquid 717 to it as illustrated at 720. When the dyed fluid 717 is drawn to the sidewall region of the electrode 727, it is moved out of the optical path and the pixel can become more transparent to light. This embodiment would therefore allow for the pixel-based control of light emanating from a Photonic Emitter to be passed on through. In some embodiments, this may allow for a projection system to be formed from a combination of an array of Photonic Emitters each with a corresponding pixel element comprising an electro-wetting on dielectric cell to control transmittance. As described herein, these embodiments may also comprise a light source, control electronics for both the light source and the pixel elements, and a lens system to focus the near field image at a desired location, which may comprise a user's retina. There may be numerous alternatives to the electro-wetting on dielectric cell that may allow for the control of the transmittance of light near a Photonic Emitter. Additionally, the example provided of the electro-wetting on dielectric based cell may have numerous alternatives including for example the reversal of the type of fluid that may comprise a dye or an inherent quality to block light.

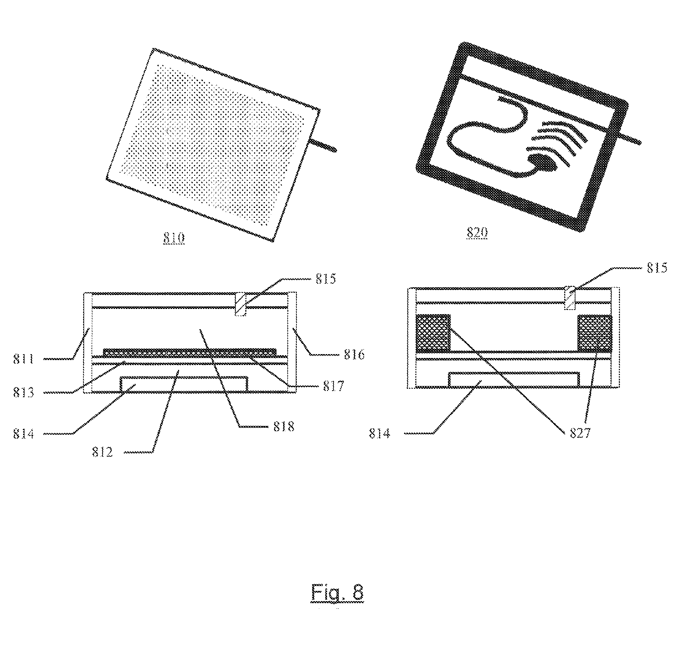

Referring now to Fig. 8, an alternative exemplary embodiment of an EWOD pixel based light intensity-modulating cell is illustrated. In the present embodiment, the electrode 814 in proximity to a surface 813 along which a fluid 817 will be attracted is not on the sidewall of a vertical structure 811 and 816 but along one of the cell faces 812. Because the device may operate with light proceeding through this surface 813, the use of relatively transparent electrodes 814 and 815 can be important in such embodiments. As mentioned in other parts of this disclosure, the use of ITO as the material for the electrode 814 and 815 can be an acceptable solution. As well, there may be modifications that allow the electrode 814 and 815 to be located on the periphery of the EWOD cell face as well. Nevertheless, in Fig. 8, a cell 810 where the light absorbing material is blocking the majority of the cell surface is illustrated. The represent a fluid 817 with an absorbing characteristic can be inherent of the fluid or results from the use of dyes. The other fluid 818 may not significantly interact with light through the cell. A surface 813 which has a defined surface free energy which may be either inherent or may result from processing designed to establish a surface characteristic. An optional layer 812 of dielectric material may be present if the surface 813 is created either as an additional film upon a dielectric or as a surface modification of a dielectric. An electrode 814 can be useful in defining the region of the dielectric surface that is affected when an electro-potential is applied across the EWOD cell. Structural light containment 811 and 816 can be used to define pixels. When an electro-potential is applied across the cell at electrodes 814 and 815, the state of the cell may be as depicted at 820. By causing the light absorbing fluid 817 to be repelled in the region of the surface above the electrode 814, the fluid can move to the edges 827 of the pixel element. Therefore, it is moved out of the optical path and the pixel can become more transparent to allow light to pass through it.

### ENERGIZED OPHTHALMIC DEVICES WITH PHOTONIC EMITTERS

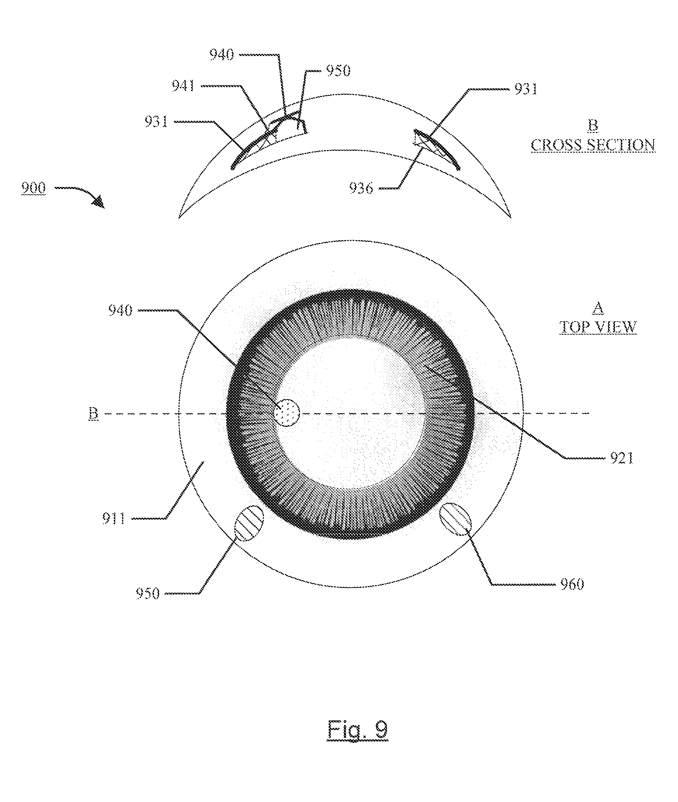

Referring now to Fig. 9, an exemplary energized ophthalmic device 900 for a projection system comprising photonic arrays, light phase or intensity modulation arrays and lens systems that may be useful for implementing some aspects of the disclosure is illustrated. The Ophthalmic Device 900 which capable of being worn on a user's eye surface. A hydrogel-based skirt 911 that completely surrounds in some embodiments, or partially surrounds or supports a Media Insert 936 device in other embodiments. In the present embodiment, the hydrogel skirt 911 can surround a fundamentally annular Media Insert 936. Sealed within the Media Insert 936 may be energization elements, electronic circuitry for control, activation, communication, processing and the like (not identified in Fig. 9). The energization elements may be single use battery elements or rechargeable elements along with power control systems, which can enable the recharging. The components may be located in the Media Insert 936 as discrete components or as stacked integrated devices with multiple active layers.

The Ophthalmic Device 900 may have structural and cosmetic aspects to it including, stabilization elements 950 and 960 which may be useful for defining orientation of the device upon the user's eye and for aligning the Ophthalmic Device 900 with the line of sight of the user appropriately. The annular Media Insert 936 may have patterns 921 and 931 printed upon one or more of its surfaces depicted as an iris pattern 921 and 931. Other patterns may be appropriate to produce holograms or visual effects which can be desired.

The Media Insert 936 may have a photonic-based imaging system 940 in a small region of the optical zone. As previously mentioned, in some embodiments a 64x64 pixel imaging system may be formed with a size roughly 0.5 mm x 0.5 mm in size. In cross section B, it may be observed that the photonic based imaging system 940 may be a photonic projection component that can comprise photonic emitter elements; an EWOD based pixel transmittance control device, a light source or multiple light sources and electronics to control these components. The photonic-based imaging system 940 may be attached to a lens system 950 and be connected to the annular Media Insert 936 by a data and power interconnection bus 941.

In some embodiments, the lens system 950 may be formed of static lens components that focus the near field image of the imaging system to a fixed location in space related to the body of the ophthalmic device 900. In other embodiments, the lens system 950 may also include active components. For example, a meniscus based lens device with multiple electrode regions may be used to both translate the center of the projected image and adjust the focal power of the device to adjust the focus, and effectively the size of the image projected. The lens device may have its own control electronics, or alternatively it may be controlled and powered by either the photonic-based imaging system 940 or the annular Media Insert 936 or both.

In some embodiments, the display may be a 64 x 64 based projection system, but more or less pixels are easily within the scope of the inventive art, which may be limited by the size of the pixel elements and the ophthalmic device itself. The display may be useful for displaying dot matrix textual data, image data or video data. The lens system may be used to expand the effective pixel size of the display in some embodiments by rastering the projection system across the user's eye while displaying data. The display may be monochromatic in nature or alternatively have a color range based on multiple light sources.

### METHODS FOR OPHTHALMIC DEVICES WITH PHOTONIC EMITTERS

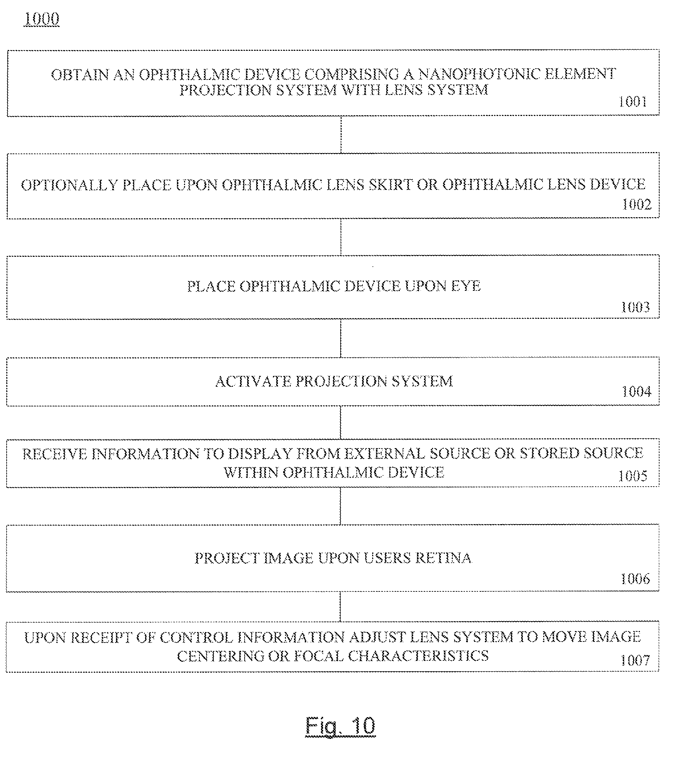

Referring now to Fig. 10, method steps related to the use of ophthalmic devices comprising Photonic Emitters according to some aspects of the disclosure are illustrated in a flowchart 1000. At step 1001, a user obtains an ophthalmic device with an attached Photonic Emitter based projection system. In connection with an array of Photonic Emitters with a corresponding pixel element comprising an electro-wetting on dielectric cell to control transmittance may be a light source, control electronics for both the light source and the pixel elements, and a lens system to focus the near field image at a desired location which may comprise a user's retina. The system may comprise electronic components, energization elements, sensors to mention examples.

At step 1002, if the ophthalmic device is not encapsulated in a hydrogel skirt, the user may attach the device to a skirt of hydrogel or place the ophthalmic device upon another lens itself.

At step 1003, the complete ophthalmic device may be placed upon the user's eye. Moreover, at step 1004 an activation signal of some kind may activate the projection system within the ophthalmic device. At step 1005, data may be received into the control for the projection system. In some cases, the data may be found within the ophthalmic device either as stored data within a memory element or data obtained by sensing elements upon the ophthalmic device. In other cases, a receiving element within the ophthalmic device may receive data from a source external to the ophthalmic device. At step 1006, the data may be projected by the ophthalmic device. The projection of the data may comprise a textual presentation, or a graphic presentation of image data or video data. At step 1007, the user may use an external controlling device to broadcast control signals to the ophthalmic device. The control signals may cause numerous operating parameters of the lens system to change. Amongst the parameters to be altered may be the focal characteristics of the lens system and the centering of the image upon the retina as well.

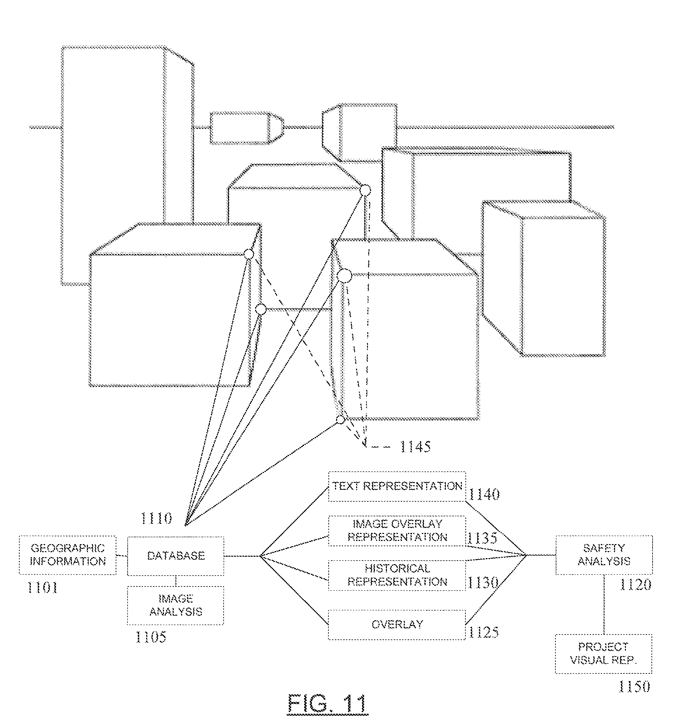

Referring now to Fig. 11, a perspective view of a geographic setting with objects useful for a sensor and a processor to associate related data to provide Visual Representations is illustrated. At 1100, four objects 1145 that may be associated with a geographic location 1101 can be identified. The geographic location 1101 information may be determined by a global positioning system, for example, of a mobile phone that is in communication with a processor in the Ophthalmic Device. The Ophthalmic Device may include a database 1110 and/or connect with a database that can associate objects in a captured image, that can be relatively easy to recognize by one or more sensor contained in the Ophthalmic Lens. The sensor may include, for example, an image sensor that can send data to a processor via a communication device to perform an image analysis 1105. The processor can be associated with the database containing Visual Representations and match the information received with data associated with the geographic setting being observed by the wearer of the Ophthalmic Lens. Visual Representations can include, for example, a display of text 1140, an image overlay representation 1135, a historical representation 1130, an overlay image 1125, and the such. These may be projected according to embodiments of the disclosure.

In a preferred embodiment, the same image sensor or a different sensor can send information to the processor in the Ophthalmic Lens to perform a safety analysis 1120. For example, the image sensor can prevent the Visual Representation from being projected when a user's eyelids are shut for more than a predetermined period of time. Other sensors can include, for example, biomarker sensors and/or neurological signal sensors that can sense the user's condition and predict how they will react to the projected Visual Representation. The prediction may be based on previously recorded data, or based on previous sensed reactions by the specific user. In addition, in some embodiments, the user may be able to access and modify settings of the system through a user's interface. The interface can include, for example, a mobile phone with an application where stored information can be accessed and modified and which is associated and in communication with the Ophthalmic Device.

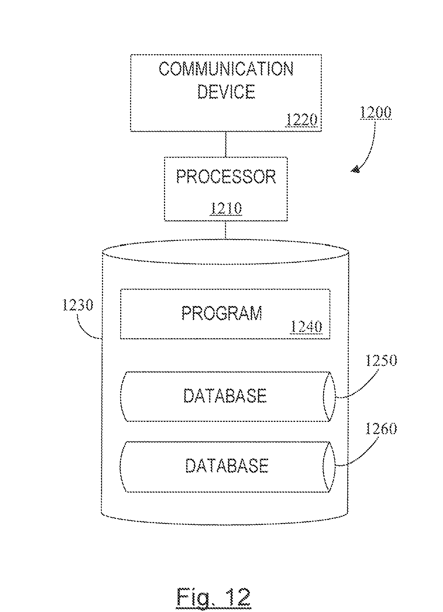

Referring now to FIG. 12, a controller 1200 that may be embodied in one or more of the above listed devices and utilized to implement some embodiments of the present disclosure. The controller 1200 comprises a processor unit 1210, such as one or more processors, coupled to a communication device 1220 configured to communicate via a communication network. The communication device 1220 may be used to communicate, for example, with one or more Bluetooth devices such as a personal computer, cellular telephone, tablet, computer, automobile, or a handheld device.

The processor 1210 can also be in communication with a storage device 1230. The storage device 1230 may comprise any appropriate information storage device, including combinations of electronic storage devices, such as, for example, one or more of: hard disk drives, optical storage devices, and semiconductor memory devices such as Random Access Memory (RAM) devices and Read Only Memory (ROM) devices.

The storage device 1230 can store a program 1240 for controlling the processor 1210. The processor 1210 performs instructions of the program 1240, and thereby operates in accordance with aspects of the disclosure. The processor 1210 may also cause the communication device 1220 to transmit information, including, in some instances, control commands to operate apparatus to implement the processes described above. Specific examples of apparatus utilized to implement various aspects of the invention can include a computer server, a personal computer, a laptop computer, a handheld computer, an iPod, a mobile phone, tablet, or other communication device, or any other processor and display equipped device.

In some preferred embodiments, apparatus can be in communication with a video and data server farm. The video and data server farm may include at least one Visual Representation associated with the location. The Visual Representation may correspond to, for example, to a geographic location, a sensed condition by the contact lens, and/or a specific time of the day. All of the Visual Representations can be correlated and displayed based on a safety assessment performed by one or more sensor associated with the Ophthalmic Device.

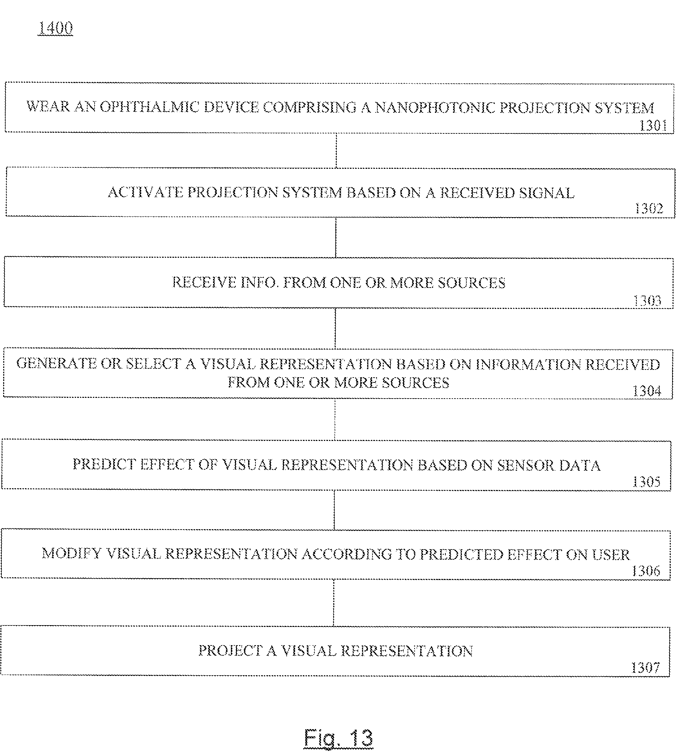

Referring now to FIG. 13, method steps related to the projection of Visual Representations according to some aspects of the disclosure are illustrated in a flow chart. At step 1301, a user wears an Ophthalmic Device comprising a nanophotonic projection system. At step 1302, the projection system may be activated based on a received signal. The signal may be from a sensor or a signal from the user requesting information about a particular setting. At step 1303, the information is received from one or more sources associated with the ophthalmic device or from a database contained in the ophthalmic device. Information contained within the ophthalmic device may be, for example, health assessment measurement thresholds.

At step 1304, the Visual Representation may be generated or pulled from one or more associated databases. At step 1305, the effect of the ready to be generated representation can be predicted/evaluated based on additional sensor data gathered and predetermined settings. For example, ambient light conditions, levels of a biomarker, lapsed time since the request for the signal of step 1302 was received and/or user's position since the request for the information. At step 1306, the Visual Representation may be modified to fit the condition or be eliminated completely. At step 1307, if a positive effect is predicted, from the original representation, and/or the modified representation, the Visual Representation can be projected by the projection system. In some preferred embodiments, the projection system can include embodiments of the Pixel Based Light Modulating Systems disclosed herein.

A non-exhaustive list of various aspects of the present invention is set out in the following numbered clauses:
Clause 1. An energized ophthalmic device, comprising:
   one or more modulated photonic emitters of the ophthalmic device;
   a media insert supporting a first processor and one or more light sources of the ophthalmic device;
   the one or more light sources configured to generate light, wherein at least a portion of the generated light from the one or more light sources is emitted by the one or more photonic emitters of the ophthalmic device;
   the first processor of the ophthalmic device configured to:
      receive, from a sensor, an indication to project a visual representation, and
      control, in response to the received indication, at least one of the one of more modulated photonic emitters and the one or more light sources based on one or more programmed parameters; and
   one or more antennas of the ophthalmic device configured to communicate with the first processor and a second processor, the second processor configured to generate the visual representation.
Clause 2. The energized ophthalmic device of clause 1, wherein the one or more modulated photonic emitters comprise a semiconductive material.
Clause 3. The energized ophthalmic device of clause 1, wherein the one or more modulated photonic emitters comprise a resistive heating element.
Clause 4. The energized ophthalmic device of clause 1, wherein the one or more light sources comprise a light emitting diode.
Clause 5. The energized ophthalmic device of clause 1, wherein the one or more light sources comprise a laser.
Clause 6. The energized ophthalmic device of clause 1, wherein the media insert further supports an energization element that is configured to power the first processor and the one or more light sources.
Clause 7. The energized ophthalmic device of clause 6, wherein the energization element comprises a plurality of stacked integrated substrate layers with electrical interconnections between them.
Clause 8. The energized ophthalmic device of clause 1, wherein the indication to project the visual representation received from the sensor comprises an indication that ambient light is sufficient to project the visual representation received from a light sensor.
Clause 9. The energized ophthalmic device of clause 1, wherein the indication to project the visual representation received from the sensor comprises the indication to project the visual representation received from a neurological sensor.
Clause 10. The energized ophthalmic device of clause 1, wherein the indication to project the visual representation received from the sensor comprises the indication to project the visual representation received from an image sensor.
Clause 11. The energized ophthalmic device of clause 10, wherein the indication to project the visual representation received from an image sensor comprises an indication that a predetermined object is visible through an optic zone of the energized ophthalmic device.
Clause 12. The energized ophthalmic device of clause 1, further comprising a pixel based light modulation system comprising a surface region having surface free energy that is altered by application of an electropotential field that spans the surface region.
Clause 13. The energized ophthalmic device of clause 1, wherein the visual representation comprises an image overlaid on a scene visible through an optic zone of the energized ophthalmic device.
Clause 14. The energized ophthalmic device of clause 1, wherein the visual representation comprises an image enhancement of the scene visible through an optic zone of the energized ophthalmic device.
Clause 15. The energized ophthalmic device of clause 14, wherein the image enhancement of the scene comprises alteration of brightness of the scene, alteration of saturation of the scene, alteration of color of the scene, alteration of contrast of the scene, alteration of sharpness of the scene, or alteration of hue of the scene.
Clause 16. The energized ophthalmic device of clause 1, wherein the visual representation comprises health assessment information for the wearer of the energized ophthalmic lens.
Clause 17. The energized ophthalmic device of clause 1, wherein the one or more light sources comprise a first solid state light emitting element configured to have a central wavelength of emission that is red, a second solid state light emitting element configured to have a central wavelength of emission that is green, and a third solid state light emitting element configured to have a central wavelength of emission that is blue.
Clause 18. The energized ophthalmic device of clause 1, further comprising a lens in an optic zone of the energized ophthalmic device.
Clause 19. The energized ophthalmic device of clause 18, wherein the lens is a variable focal length lens.
Clause 20. The energized ophthalmic lens device of clause 1, wherein the sensor is located outside of the energized ophthalmic lens device, and the first processor is further configured to control a communication protocol for wireless communication with the sensor.

## Claims

1. An energized ophthalmic device, comprising:
one or more modulated photonic emitters of the ophthalmic device;
a media insert supporting a first processor and one or more light sources of the ophthalmic device;
the one or more light sources configured to generate light, wherein at least a portion of the generated light from the one or more light sources is emitted by the one or more photonic emitters of the ophthalmic device;
the first processor of the ophthalmic device configured to:
receive, from a sensor, an indication to project a visual representation, and
control, in response to the received indication, at least one of the one of more modulated photonic emitters and the one or more light sources based on one or more programmed parameters; and
one or more antennas of the ophthalmic device configured to communicate with the first processor and a second processor, the second processor configured to generate the visual representation.

2. The energized ophthalmic device of claim 1, wherein the one or more modulated photonic emitters comprise a semiconductive material, or the one or more modulated photonic emitters comprise a resistive heating element.

3. The energized ophthalmic device of claim 1 or claim 2, wherein the one or more light sources comprise a light emitting diode, or the one or more light sources comprise a laser.

4. The energized ophthalmic device of any of the preceding claims, wherein the media insert further supports an energization element that is configured to power the first processor and the one or more light sources.

5. The energized ophthalmic device of claim 4, wherein the energization element comprises a plurality of stacked integrated substrate layers with electrical interconnections between them.

6. The energized ophthalmic device of any of the preceding claims, wherein the indication to project the visual representation received from the sensor comprises an indication that ambient light is sufficient to project the visual representation received from a light sensor.

7. The energized ophthalmic device of any of claims 1 to 5, wherein the indication to project the visual representation received from the sensor comprises the indication to project the visual representation received from a neurological sensor.

8. The energized ophthalmic device of any of claims 1 to 5, wherein the indication to project the visual representation received from the sensor comprises the indication to project the visual representation received from an image sensor.

9. The energized ophthalmic device of claim 8, wherein the indication to project the visual representation received from an image sensor comprises an indication that a predetermined object is visible through an optic zone of the energized ophthalmic device.

10. The energized ophthalmic device of any of the preceding claims, further comprising a pixel based light modulation system comprising a surface region having surface free energy that is altered by application of an electropotential field that spans the surface region.

11. The energized ophthalmic device of any of the preceding claims, wherein the visual representation comprises an image overlaid on a scene visible through an optic zone of the energized ophthalmic device.

12. The energized ophthalmic device of any of claims 1 to 10, wherein the visual representation comprises an image enhancement of the scene visible through an optic zone of the energized ophthalmic device.

13. The energized ophthalmic device of claim 12, wherein the image enhancement of the scene comprises alteration of brightness of the scene, alteration of saturation of the scene, alteration of color of the scene, alteration of contrast of the scene, alteration of sharpness of the scene, or alteration of hue of the scene.

14. The energized ophthalmic device of any of the preceding claims, wherein the visual representation comprises health assessment information for the wearer of the energized ophthalmic lens.

15. The energized ophthalmic device of any of the preceding claims, wherein the one or more light sources comprise a first solid state light emitting element configured to have a central wavelength of emission that is red, a second solid state light emitting element configured to have a central wavelength of emission that is green, and a third solid state light emitting element configured to have a central wavelength of emission that is blue.

16. The energized ophthalmic device of any of the preceding claims, further comprising a lens in an optic zone of the energized ophthalmic device, and preferably wherein the lens is a variable focal length lens.

17. The energized ophthalmic lens device of any of the preceding claims, wherein the sensor is located outside of the energized ophthalmic lens device, and the first processor is further configured to control a communication protocol for wireless communication with the sensor.
